# EUROPEAN PATENT APPLICATION

(11) **EP 4 776 199 A1**
(43) Date of publication of application: **15.07.2026**
(21) Application number: 24862389.4
(22) Date of filing: 24.05.2024
(51) Int. Cl.: G06Q 10/06

(54) **OUTPUT PROGRAM, OUTPUT METHOD, AND INFORMATION PROCESSING DEVICE**

(30) Priority: 08.09.2023 WO PCT/JP2023/032926
(71) Applicant: FUJITSU LIMITED, Kawasaki-shi, Kanagawa 211-8588 (JP)
(72) Inventor: NAKAYAMA, Sayuri, Kawasaki-shi, Kanagawa 211-8588 (JP); SASAMOTO, Yuki, Kawasaki-shi, Kanagawa 211-8588 (JP); TADA, Atsuko, Kawasaki-shi, Kanagawa 211-8588 (JP); INOMATA, Akihiro, Kawasaki-shi, Kanagawa 211-8588 (JP)
(74) Representative: Hoffmann Eitle
(86) International application number: PCT/JP2024/019210
(87) International publication number: WO 2025/052732

(57) **Abstract**

An output program causes a computer to execute processing of: receiving information related to a policy; identifying a policy model composed of a plurality of components that prescribes content related to the policy in a hierarchical structure using the received information related to the policy; generating a policy model in which one or more components corresponding to the information related to the policy are changed among a plurality of components possessed by the identified policy model; and outputting the generated policy model.

## Description

### Technical Field

The present invention relates to an output program, an output method, and an information processing apparatus.

### Background Art

As one of workflows, a policy flow graph is known in which a flow of assigning objects that become targets of policies in various fields such as medical care, nursing care, and administration, for example humans, etc., to services, etc. for achieving objectives of the policies is diagrammed.

For example, for generation of a policy flow graph, an AI (Artificial Intelligence) chat service realized by large-scale language models such as Transformer, etc., so-called LLM (Large Language Models) can be utilized.

Among such AI chat services, there exist those that output a flowchart with text as input. For example, by giving a prompt that sets a role such as an interviewer, etc. in which preconditions, detailed conditions, etc. are defined, it is possible to cause a flow to be output through an interview format.

### Citation List

### Patent Literature

Patent Document 1: Japanese Laid-open Patent Publication No. 2003-228647

### Summary of invention

### Technical Problem

However, the above AI chat services face challenges in generating policy flow graphs that are suited to the actual field where the policy is applied. For example, the above AI chat services may output a policy flow graph that lacks components such as services, conditions, etc. that are essential in the field, or includes components such as services, conditional branches, etc. that do not conform to standards such as laws, guidelines, etc.

Note that the above problem is not limited to policy models in flow format such as policy flow graphs, but similarly occurs across policy models in general, including those in formats such as social concepts, organizational charts, and guidelines.

In one aspect, an object is to provide an output program, an output method, and an information processing apparatus capable of realizing generation of a policy model suited to the actual field where the policy is applied. Solution to Problem

According to an aspect of an embodiment, an output program causes a computer to execute processing of: receiving information related to a policy; identifying a policy model composed of a plurality of components that prescribes content related to the policy in a hierarchical structure using the received information related to the policy; generating a policy model in which one or more components corresponding to the information related to the policy are changed among a plurality of components possessed by the identified policy model; and outputting the generated policy model.

### Advantageous Effects of Invention

According to one embodiment, generation of a policy model suited to the actual field where the policy is applied can be realized.

### Brief Description of Drawings

FIG. 1 is a block diagram illustrating an example of functional configuration of a server apparatus.
FIG. 2 is a diagram illustrating a policy flow graph.
FIG. 3 is a diagram illustrating a specific example of a policy flow graph.
FIG. 4 is a diagram illustrating an example of question collection data.
FIG. 5 is a diagram illustrating an example of flow collection data.
FIG. 6 is a schematic diagram explaining an example of association information.
FIG. 7 is a diagram (1) illustrating an example of a question window.
FIG. 8 is a diagram (2) illustrating an example of a question window.
FIG. 9 is a diagram (3) illustrating an example of a question window.
FIG. 10 is a diagram (4) illustrating an example of a question window.
FIG. 11 is a diagram (5) illustrating an example of a question window.
FIG. 12 is a diagram illustrating an example of an answer confirmation window.
FIG. 13 is a diagram illustrating an example of a policy flow generation result.
FIG. 14 is a flowchart illustrating a procedure of model output processing.
FIG. 15 is a block diagram illustrating an example of functional configuration of a server apparatus according to an application example.
FIG. 16 is a diagram (1) illustrating an example of a chatbot screen.
FIG. 17 is a diagram (2) illustrating an example of a chatbot screen.
FIG. 18 is a diagram illustrating an example of hardware configuration.
FIG. 19 is a schematic diagram illustrating an operation example of a model output function according to Example 3.
FIG. 20 is a block diagram illustrating an example of functional configuration of a server apparatus according to Example 3.
FIG. 21 is a flowchart illustrating a procedure of model output processing according to Example 3. Description of Embodiments

Hereinafter, embodiments of an output program, an output method, and an information processing apparatus according to the present application will be described with reference to the accompanying drawings. Each embodiment merely illustrates examples, and the configuration, structure, functions, numerical values used for specifying these, usage scenes, and the like are not limited by such illustrations. And, each embodiment can be adaptively combined within a range that does not contradict the processing content.

### <First Embodiment>

### <System Configuration>

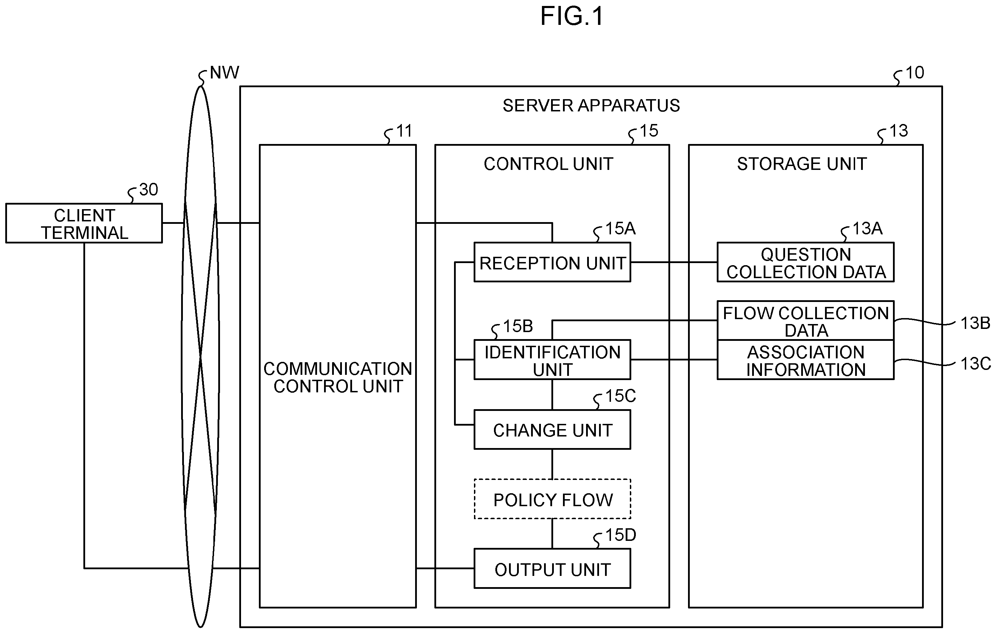

FIG. 1 is a block diagram illustrating an example of functional configuration of the server apparatus 10. The server apparatus 10 illustrated in FIG. 1 provides, from an aspect of supporting policy formulation, a model output function that outputs a policy model in which a design philosophy of a policy, namely a scenario, is digitized.

The server apparatus 10 can provide the above-mentioned model output function as a cloud service by executing PaaS (Platform as a Service) type middleware or SaaS (Software as a Service) type applications.

As illustrated in FIG. 1, the server apparatus 10 can be communicably connected to a client terminal 30 via a network NW. For example, the network NW may be any type of communication network such as the Internet or a LAN (Local Area Network), regardless of whether wired or wireless. Note that although FIG. 1 illustrates an example in which one client terminal 30 is connected to one server apparatus 10, it does not prevent an arbitrary number of client terminals 30 from being connected.

The client terminal 30 is a terminal device that receives provision of the above-mentioned model output function. For example, the client terminal 30 can be used by customers such as policy planners as an example of related parties such as local governments who are entities implementing policies. As an example, the client terminal 30 may be realized by any computer including personal computers, smartphones, tablet terminals, wearable terminals, and the like.

Note that although an example was given here in which the above-mentioned model output function is provided as a cloud service, it is not limited to this. For example, the above-mentioned model output function may be provided on-premises. Also, although an example was given in which the above-mentioned model output function is provided in a client-server system, it is not limited to this. For example, the above-mentioned model output function may be provided standalone by an application operating on the client terminal 30 causing the client terminal 30 to execute processing corresponding to the above-mentioned model output function.

### <Policy Model>

Here, a "policy model" includes a diagram composed of a plurality of components that prescribes content related to a policy in a hierarchical structure. For example, a policy model can be realized as a policy flow graph, social concepts, organizational charts, medical guidelines, and the like.

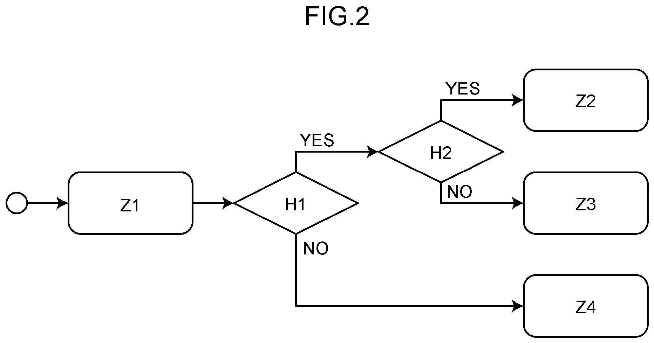

Hereinafter, as one example of a policy model, explanation will be given using a policy flow graph as an example. FIG. 2 is a diagram illustrating a policy flow graph. Z1, Z2, Z3, and Z4 illustrated in FIG. 2 indicate, for example, services that an administrator implements for users. These may also be called "service implementation components". As specific examples of services, for example, in the case of the medical field, in addition to "interventions" to which objects that become targets of policies, for example residents, etc. are assigned, such as receiving health examinations, examinations by specialists, etc., "no intervention" such as follow-up observation, etc. can be cited, but are not limited to policies in the medical field.

H1 and H2 indicate, for example, conditional branches including conditions. These may also be called "conditional branch components". As specific examples of conditions, for example, in the case of the medical field, estimated Glomerular Filtration Rate (eGFR) being less than a threshold, Hemoglobin A1c value (HbA1c) being less than a threshold, urine protein value being a threshold or more, etc. can be cited, but are not limited to conditions in the medical field.

Z1, Z2, Z3, Z4 and H1, H2 may each be called a "component". Such "components" can correspond to an example of a "node" from the aspect of graph data. Also, connections between nodes can correspond to an example of an "edge" including a "directed edge" or the like.

Note that in this embodiment, policy formulation in the medical field is described as an example, but the use case of the above-mentioned model output function is not limited to this. For example, the above-mentioned model output function may be applied to policy formulation in various fields such as the transportation field including road pricing, the energy field including decarbonization and power supply, as well as work, tests, surveys, etc. In those cases as well, the same operational effects as when applied to policy formulation in the medical field can be obtained.

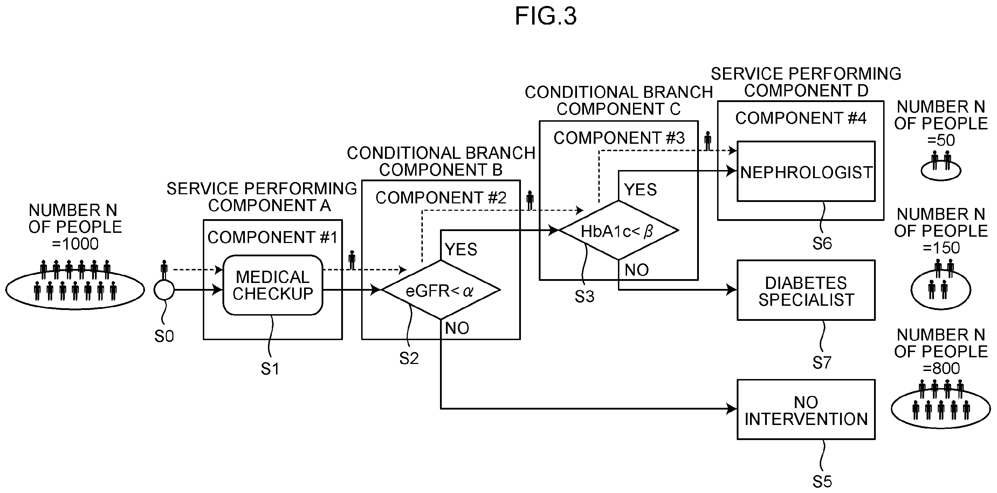

FIG. 3 is a diagram illustrating a specific example of a policy flow graph. As illustrated in FIG. 3, a policy is modeled as a workflow composed of combinations of components such as conditional branches and service implementations. Then, the number of persons receiving each service is output from a model that has accumulated and trained information on the manner of flow of persons and parameters from actual values when used for each conditional branch component.

In the example illustrated in FIG. 3, at reference sign S0, the number of persons N=1000 is input. At reference sign S1, component #1 as service implementation component A is set to "health examination". At reference sign S2, component #2 as conditional branch component B is set to "eGFR < α". When "eGFR<α" is not satisfied (see NO route at reference sign S2), as illustrated at reference sign S5, it is determined that there is "no intervention" by a specialist for that citizen.

On the other hand, when "eGFR<α" is satisfied (see YES route at reference sign S2), as illustrated at reference sign S3, component #3 as conditional branch component C is set to "HbA1c<β". When "HbA1c < β" is satisfied (see YES route of reference sign S3), as illustrated by reference sign S6, component #4 as conditional branch component D is set to "kidney specialist", and it is determined that intervention of "kidney specialist" to the citizen concerned is necessary. On the other hand, when "HbA1c<β" is not satisfied (see NO route at reference sign S3), as illustrated at reference sign S7, it is determined that intervention by a "diabetes specialist" is necessary for that citizen.

In the example illustrated in FIG. 3, as illustrated by arrows, the number of persons flowing in the order of component #1, component #2, component #3, and component #4 is predicted. For example, the result of assigning the number of persons N=1000 to Z2 to Z4 which are interventions in the policy flow graph illustrated in FIG. 3 is as follows. 50 persons are assigned to intervention Z2. 150 persons are assigned to intervention Z3. Furthermore, 800 persons are assigned to intervention Z4.

Hereinafter, the policy flow graph may be abbreviated as "policy flow" by referring to it. Policy flows may be shared in any framework. As just one example, policy flows can be shared among organizations around the world, for example public organizations such as local governments, through a platform of a data infrastructure in which sharing, mutual reference, and updating of policy flows are possible.

Such a data infrastructure platform may be provided by a business operator providing the above-mentioned model output function, or may be provided by another business operator. For example, a policy planner can refer to policy flows from around the world collected in the above-mentioned data infrastructure via the client terminal 30. At this time, by incorporating all or a part of policy flows collected in the data infrastructure, existing policy flows can be updated to support formulation of a draft policy flow.

In addition, the above-mentioned data infrastructure can provide not only sharing of policy flows but also the following backend functions. For example, a simulation that simulates a flow of objects targeted as services on existing and draft policy flows can be executed. In addition, indicators of each of existing and draft policy flows, for example effects, costs, etc. can be evaluated, or comparison between existing and draft policy flows, or comparison between a plurality of draft policy flows can be executed.

### <Configuration of Server Apparatus 10>

Next, the functional configuration of the server apparatus 10 that provides the above-mentioned model output function will be described. FIG. 1 schematically illustrates blocks related to the model output function possessed by the server apparatus 10. As illustrated in FIG. 1, the server apparatus 10 has a communication control unit 11, a storage unit 13, and a control unit 15. Note that FIG. 1 merely illustrates functional units related to the above-mentioned model output function in an extracted manner, and functional units other than those illustrated may be provided in the server apparatus 10.

The communication control unit 11 is a functional unit that controls communication with other devices such as the client terminal 30. As just one example, the communication control unit 11 can be realized by a network interface card such as a LAN card. As one aspect, the communication control unit 11 receives a generation request for a policy flow from the client terminal 30, or outputs a policy flow to the client terminal 30.

The storage unit 13 is a functional unit that stores various types of data. As just one example, the storage unit 13 may be realized by internal, external, or auxiliary storage of the server apparatus 10. For example, the storage unit 13 stores question collection data 13A, flow collection data 13B, and association information 13C. Note that the question collection data 13A, flow collection data 13B, and association information 13C are merely examples, and it does not prevent other data besides these from being stored in the storage unit 13.

The question collection data 13A may be a set of question templates in which questions related to policies are typified. Among such question templates, a question that accepts designation of a policy type to be formulated may be included among a set of flow templates in which basic forms of policy flows are typified for each policy type. In addition to this, a question that customizes a part of the flow template may be included in the question templates.

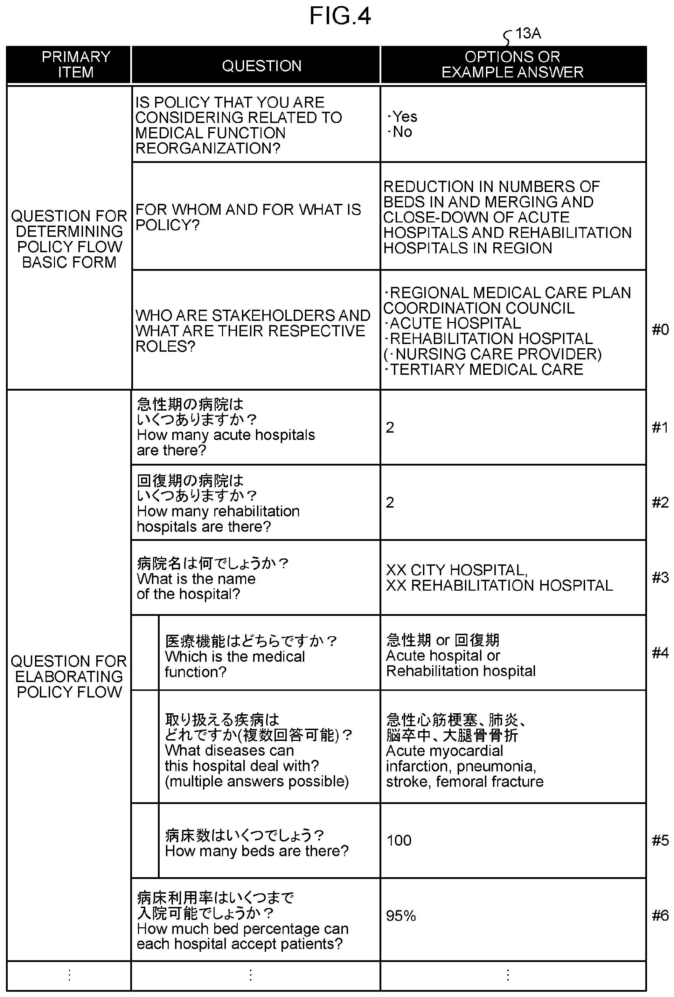

FIG. 4 is a diagram illustrating an example of the question collection data 13A. As illustrated in FIG. 4, the question collection data 13A may be data in which items such as "Major Item", "Question", and "Options or Answer Examples" are associated.

The "Major Item" mentioned here refers to an item that categorizes questions. Also, "Question" refers to text corresponding to a question or the definition of a question. Also, "Options or Answer Examples" refers to answer options or answer examples for the question. Note that while "Options or Answer Examples" is illustrated for convenience of explanation, it does not necessarily have to be used for presenting questions or obtaining answers.

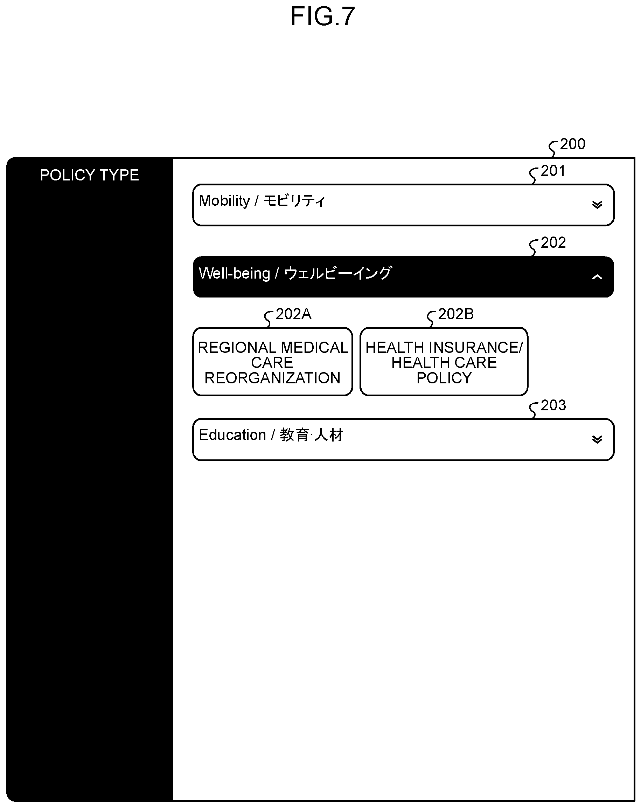

For example, FIG. 4 illustrates an excerpt of a question template collection related to medical function reorganization as just one example of a policy type. Details will be described later using FIGS. 7 to 12, but by presenting questions to the client terminal 30 based on the question templates illustrated in FIG. 4, designation such as a policy type that identifies the type of policy to be formulated or content that customizes the flow template, etc. can be accepted as answers.

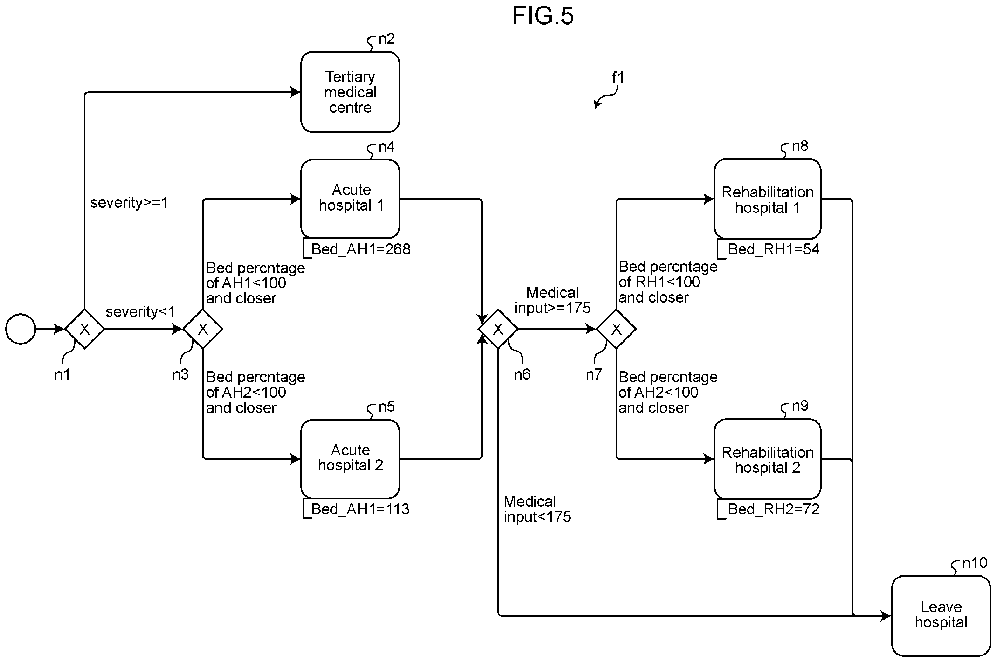

The flow collection data 13B may be a set of flow templates in which basic forms of policy flows are typified. FIG. 5 is a diagram illustrating an example of the flow collection data 13B. FIG. 5 illustrates an excerpt of a flow template f1 in which the basic form of a policy flow of the policy type "medical function reorganization" is typified as just one example of a flow template. As illustrated in FIG. 5, in the flow template f1, a procedure of allocating objects such as residents, etc. to service components corresponding to each medical function in the order of tertiary emergency, acute phase, and rehabilitation phase is described in BPMN (Business Process Model and Notation) format.

The association information 13C is information in which the association between question templates and flow templates is defined. As just one example, in the association information 13C, an association between a question template and target elements that are customized according to an answer corresponding to the question template among flow templates, for example components such as services, conditional branches, etc. or parameters possessed by components may be described. Such associations may be described in any format such as JSON (Java (registered trademark) Script Object Notation), markup languages, or the like, as just one example.

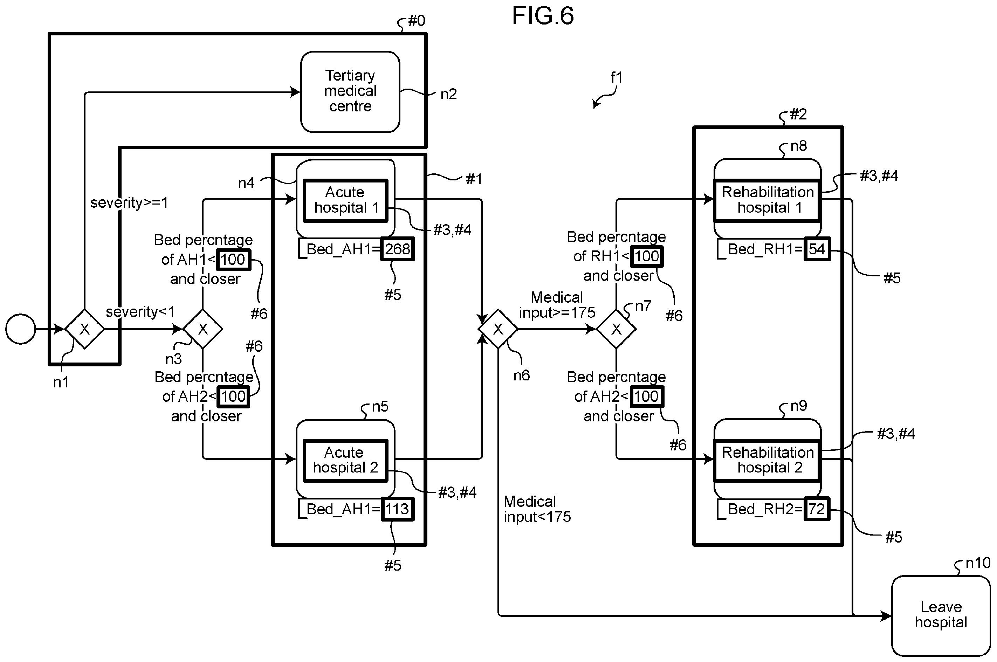

FIG. 6 is a schematic diagram explaining an example of the association information 13C. FIG. 6 illustrates related elements #0 to #6 that are related to the question templates included in the question collection data 13A illustrated in FIG. 4 among the flow template f1 illustrated in FIG. 5, indicated by thick-line frames.

The association information 13C may include the related elements #0 to #6 illustrated in FIG. 6. Furthermore, information of modification patterns that customize a part of the flow template according to answers corresponding to question templates may be associated with related elements #0 to #6. Such modification patterns may include, as just one example, the following four modification patterns P1 to P4.

As one aspect, a modification pattern P1 for customizing service components or parameters possessed by the components arranged in parallel on the flow template is linked to related elements #1 to #4.

For example, in the case of flow template f1, acute medical institutions and rehabilitation medical institutions are mentioned as examples of service components arranged in parallel on flow template f1. In this flow template f1, two components n4 and n5 are exemplified as examples of acute medical institutions, and two components n4 and n5 are exemplified as examples of rehabilitation medical institutions, but the number of these medical institutions can be changed according to modification pattern P1.

Specifically, in the example of related element #1, the number of acute medical institutions arranged in parallel in flow template f1 is changed according to the answer corresponding to the question template of related element #1. Also, in the example of related element #2, the number of rehabilitation medical institutions arranged in parallel in flow template f1 is changed according to the answer corresponding to the question template of related element #2.

Also, in flow template f1, examples of parameters possessed by service components arranged in parallel on flow template f1 include the names and medical functions of the medical institutions. Such parameters can also be changed according to modification pattern P1.

Specifically, in the case of the example of related element #3, the names of acute medical institutions n4 and n5 or the names of rehabilitation medical institutions n8 and n9 are set according to the answer corresponding to the question template of related element #3. Also, in the example of related element #4, the medical functions of medical institutions n4, n5, n8, and n9, that is, acute phase or rehabilitation phase, are selected according to the answer corresponding to the question template of related element #4.

As another aspect, a modification pattern P2 that changes meta information of service components included in the flow template is associated with related element #5. For example, in the case of the example of related element #5, as an example of detailed information of medical institutions n4, n5, n8, and n9, the number of beds is set according to the answer corresponding to the question template of related element #5. Note that the number of beds is merely one example of an item included as detailed information of medical institutions, and there may be other items such as the number of physicians.

As a further aspect, a modification pattern P3 that changes parameters of conditional branch components included in the flow template is associated with related element #6. For example, in the example of related element #6, the threshold value of the bed utilization rate used in the determination of conditional branch component n3 and conditional branch component n7 is set according to the answer corresponding to the question template of related element #6.

As another aspect, a modification pattern P4 that deletes conditional branch components included in the flow template, or such conditional branch components and service components connected thereto, is associated with related element #0. For example, in the case of the example of related element #0, conditional branch component n1 and a service connected to conditional branch component n1, namely tertiary emergency component n2 are set according to the answer corresponding to the question template of related element #0.

Thus, in the case of the example illustrated in FIG. 6, the association information 13C may include related element #0 (modification pattern P4), related elements #1 to #4 (modification pattern P1), related element #5 (modification pattern P2), and related element #6 (modification pattern P3), etc.

The control unit 15 is a functional unit that performs overall control of the server apparatus 10. As just one example, the control unit 15 can be realized by a hardware processor. In addition, the control unit 15 may be realized by hard-wired logic. As illustrated in FIG. 1, the control unit 15 has a reception unit 15A, a identification unit 15B, a change unit 15C, and an output unit 15D.

The reception unit 15A is a processing unit that receives various types of information from the client terminal 30. As just one example, the reception unit 15A can receive a policy flow generation request from the client terminal 30. When such a policy flow generation request is received, the reception unit 15A can receive various types of information related to the policy to be formulated.

As one aspect, the reception unit 15A presents to the client terminal 30 a question that imposes an answer of a flow template, a so-called type of flow.

FIG. 7 is a diagram (1) illustrating an example of a question window. FIG. 7 illustrates a question window 200 generated according to question templates belonging to the major item "Questions for determining the basic form of policy flow" among the question collection data 13A illustrated in FIG. 4.

As illustrated in FIG. 7, the question window 200 includes pull-down menus 201, 202, and 206. These pull-down menus 201, 202, and 206 correspond to major categories of policy type "Mobility", "Well-being", and "Education/Human Resources".

At this time, when an operation is performed on any of the pull-down menus 201, 202, and 206, item buttons corresponding to the selected pull-down menu are displayed.

As just one example, FIG. 7 illustrates an example in which pull-down menu 202 is selected. In this case, item buttons 202B and 202C corresponding to the pull-down menu are displayed. These item buttons 202B and 202C correspond to policy type subcategories: "Regional Medical Reorganization" and "Health Insurance Health Measures". By accepting an operation on either of these item buttons 202B and 202C, designation of the policy type can be accepted.

As another aspect, the reception unit 15A presents to the client terminal 30 a question that imposes an answer of content that customizes a flow template for which designation was accepted from the client terminal 30.

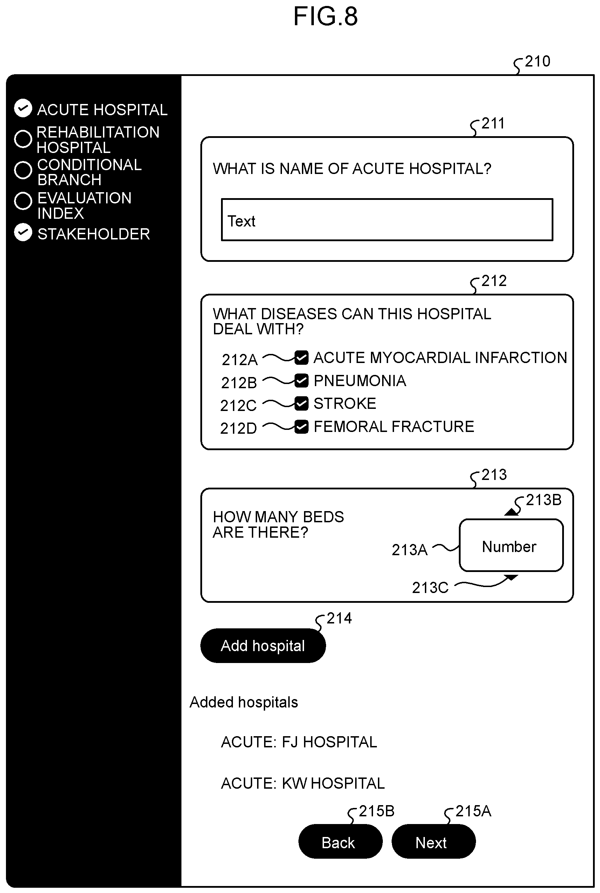

FIG. 8 is a diagram (2) illustrating an example of a question window. FIG. 8 illustrates a question window 210 generated according to question templates belonging to the major item "Questions for refining the policy flow" among the question collection data 13A illustrated in FIG. 4.

As just one example, the question window 210 may transition from the question window 200 when an operation is performed on the item button 202B for the policy type "Regional Medical Reorganization" in the question window 200.

As illustrated in FIG. 8, in the question window 210, question display fields 211 to question display fields 213 that include questions regarding customization related to acute hospitals, in other words processing, editing, etc. are displayed.

Among these, question display field 211 is generated based on the question template "What is the hospital name?" from the question collection data 13A illustrated in FIG. 4. Furthermore, question display field 211 includes a text box 211A. Through text input into such text box 211A, an answer for the name of the acute hospital can be accepted.

Also, question display field 212 is generated based on the question template "Which diseases can be handled (multiple answers possible)?" from the question collection data 13A illustrated in FIG. 4. Furthermore, question display field 212 includes four checkboxes 212A to 212D. Through selection operations on these checkboxes 212A to 212D, answers for one or multiple types of diseases that the acute hospital can handle can be accepted.

Furthermore, question display field 213 is generated based on the question template "How many beds are there?" from the question collection data 13A illustrated in FIG. 4. Furthermore, question display field 213 includes a text box 213A that accepts numerical input, a spin button 213B that accepts increases in numerical values, and a spin button 213C that accepts decreases in numerical values. Through a spin box including this text box 213A, spin button 213B, and spin button 213C, an answer for the number of beds in the acute hospital can be accepted.

In a state where answers to these question display fields 211 to 213 have been input, when an operation is executed on a GUI button 214 "Add hospital", a request to add an acute hospital corresponding to the answers to question display fields 211 to 213 as a customization target of the flow template is accepted.

As just one example, FIG. 8 illustrates an example in which "FJ Hospital" and "KW Hospital" are added as examples of customization targets of the flow template. When an operation is performed on a GUI button 215A "Next" in a state where the customization targets of the flow template have been registered in this way, transition to the presentation of the next window occurs. Note that when an operation is performed on a GUI button 215B "Back", return to the presentation of the previous window occurs.

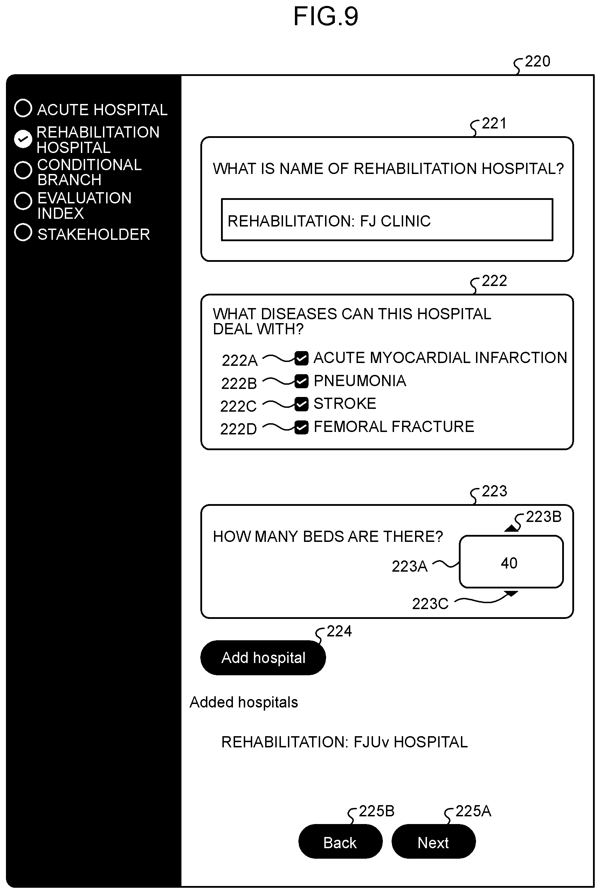

FIG. 9 is a diagram (3) illustrating an example of a question window. FIG. 9 illustrates a question window 220 generated according to question templates belonging to the major item "Questions for refining the policy flow" among the question collection data 13A illustrated in FIG. 4.

As illustrated in FIG. 9, the question window 220 displays question display fields 221 to 223 that include questions about customization related to rehabilitation hospitals, in other words, questions about processing, editing, etc.

Among these, question display field 221 is generated based on the question template "What is the hospital name?" from the question collection data 13A illustrated in FIG. 4. Furthermore, question display field 221 includes a text box 221A. Through text input into such text box 221A, an answer for the name of the rehabilitation hospital can be accepted.

Also, question display field 222 is generated based on the question template "Which diseases can be handled (multiple answers possible)?" from the question collection data 13A illustrated in FIG. 4. Furthermore, question display field 222 includes four checkboxes 222A to 222D. Through selection operations on these checkboxes 222A to 222D, answers for one or multiple types of diseases that the rehabilitation hospital can handle can be accepted.

Furthermore, question display field 223 is generated based on the question template "How many beds are there?" from the question collection data 13A illustrated in FIG. 4. Furthermore, question display field 223 includes a text box 223A that accepts numerical input, a spin button 223B that accepts increases in numerical values, and a spin button 223C that accepts decreases in numerical values. Through a spin box including this text box 223A, spin button 223B, and spin button 223C, an answer for the number of beds in the rehabilitation hospital can be accepted.

When an operation is performed on the GUI button 224 "Add hospital" in a state where answers have been input to these question display fields 221 to 223, a request to add a rehabilitation hospital corresponding to the answers to question display fields 221 to 223 as a customization target of the flow template is accepted.

As just one example, FIG. 9 illustrates an example in which "FJUv Hospital" and "FJ Clinic" are added as examples of customization targets of the flow template. When an operation is performed on a GUI button 225A "Next" in a state where the customization targets of the flow template have been registered in this way, transition to the presentation of the next window occurs. Note that when an operation is performed on a GUI button 225B "Back", return to the presentation of the previous window occurs.

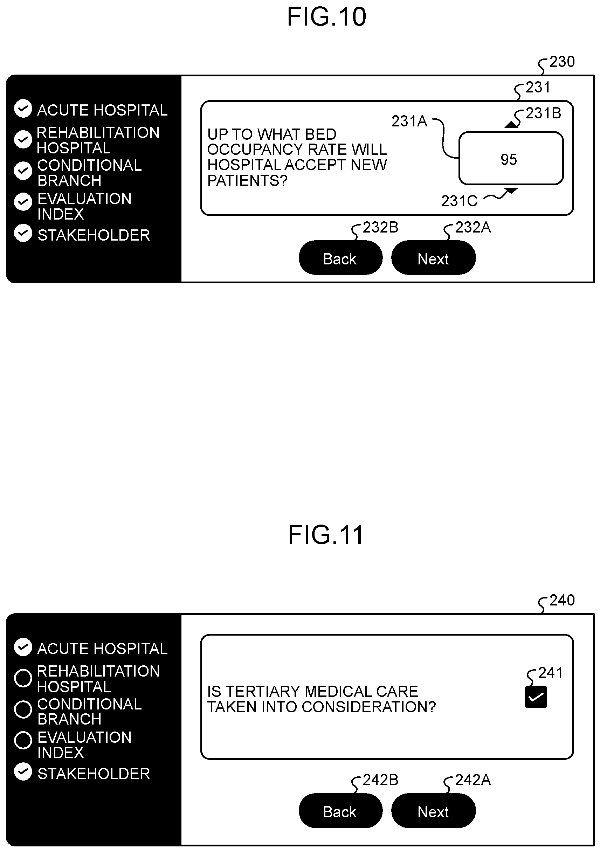

FIG. 10 is a diagram (4) illustrating an example of a question window. FIG. 10 also illustrates a question window 230 generated according to question templates belonging to the major item "Questions for refining the policy flow" among the question collection data 13A illustrated in FIG. 4.

As just one example, the question window 230 may transition from the question window 220 when an operation is performed on the GUI button 225A "Next" in the question window 220.

As illustrated in FIG. 10, in the question window 230, a question display field 231 that includes a question imposing an answer of a bed utilization rate at which new patients can be accepted at each medical institution of acute hospitals and rehabilitation hospitals is displayed. Such question display field 231 is generated based on the question template "Up to what bed utilization rate can patients be admitted?" from the question collection data 13A illustrated in FIG. 4.

Furthermore, question display field 231 includes a text box 231A that accepts numerical input, a spin button 231B that accepts increases in numerical values, and a spin button 231C that accepts decreases in numerical values.

An answer of a bed utilization rate at which new patients can be accepted can be accepted through a spin box including this text box 231A, spin button 231B, and spin button 231C.

In a state where an answer to the question display field 231 has been input in this way, when an operation is executed on a GUI button 232A "Next", transition to presentation of the next window occurs. Note that when an operation is performed on a GUI button 232B "Back", return to the presentation of the previous window occurs.

Note that although FIG. 10 cites an example of accepting an answer of a bed utilization rate at which new patients can be accepted commonly for the two medical functions of acute hospitals and rehabilitation hospitals, it is not limited thereto. For example, an answer of a bed utilization rate at which new patients can be accepted at acute hospitals and an answer of a bed utilization rate at which new patients can be accepted at rehabilitation hospitals may be accepted individually.

FIG. 11 is a diagram (5) illustrating an example of a question window. FIG. 11 illustrates a question window 240 generated according to question templates belonging to the major item "Questions for determining the basic form of policy flow" among the question collection data 13A illustrated in FIG. 4.

As just one example, the question window 240 may transition from the question window 230 when an operation is performed on the GUI button 232A "Next" in the question window 230. This is just one example, the order in which the question window 240 is displayed is not limited to next to the question window 230, and may be transitioned next to the question window 200.

As illustrated in FIG. 11, in the question window 240, a question display field 241 that includes a question imposing an answer as to whether or not to make tertiary emergency within the category of the policy is displayed. Such question display field 241 is generated based on the question template "What are the stakeholders and their respective roles?" from the question collection data 13A illustrated in FIG. 4.

Furthermore, question display field 241 includes a checkbox 241A. An answer of necessity or non-necessity of the service "tertiary emergency" component n2 and the conditional branch component n1 that allocates objects to the service "tertiary emergency" component n2 can be accepted through presence or absence of a check on such checkbox 241A.

Then, when an operation is performed on a GUI button 242A "Next", transition to the presentation of the next window occurs. Note that when an operation is performed on a GUI button 242B "Back", return to the presentation of the previous window occurs.

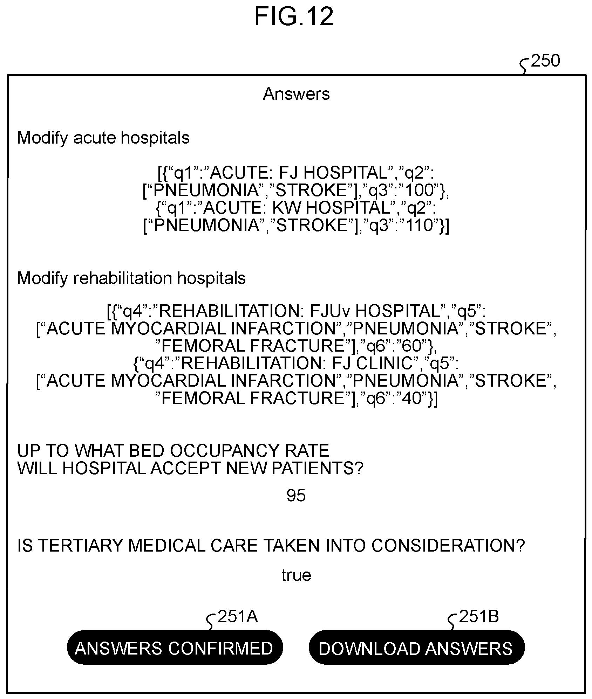

FIG. 12 is a diagram illustrating an example of an answer confirmation window. As illustrated in FIG. 12, in the answer confirmation window 250, a confirmation display of the input results of answers in the question windows 200 to 240 illustrated in FIGS. 7 to 11 is executed.

According to this answer confirmation window 250, a confirmation display to the effect that answers to add "FJ Hospital" and "KW Hospital" as customization targets for acute hospitals have been accepted is performed. Furthermore, as an example of detailed information of the acute hospital "FJ Hospital", a confirmation display to the effect that answers of types of diseases that can be handled "pneumonia" and "stroke" have been accepted together with an answer of the number of beds "100" is performed. Also, as an example of detailed information for the acute hospital "KW Hospital", a confirmation display is made that answers for the types of diseases that can be handled, "pneumonia" and "stroke", have been accepted, along with an answer for the number of beds "110".

Furthermore, according to the answer confirmation window 250, a confirmation display is made that answers to add "FJUv Hospital" and "FJ Clinic" as customization targets for rehabilitation hospitals have been accepted. Furthermore, as an example of detailed information for the rehabilitation hospital "FJUv Hospital", a confirmation display is made that answers for the types of diseases that can be handled, "acute myocardial infarction", "pneumonia", "stroke", and "femoral fracture", have been accepted, along with an answer for the number of beds "60". Also, as an example of detailed information of the acute hospital "FJ Clinic", a confirmation display indicating that answers of types of diseases that can be handled ("acute myocardial infarction", "pneumonia", "stroke", and "femoral fracture") and the number of beds ("40") have been accepted is performed.

Also, according to the answer confirmation window 250, a confirmation display to the effect that an answer of a bed utilization rate "95%" at which new patients can be accepted commonly for the two medical functions of acute hospitals and rehabilitation hospitals has been accepted as a customization target for parameters of conditional branches is performed.

Furthermore, according to the answer confirmation window 250, a confirmation display is made that as a customization target for stakeholders, an answer of "true" for the necessity of considering tertiary emergency has been accepted.

Here, when an operation is performed on a GUI button 251A "Confirm Answers", customization of the flow template is started. Note that when an operation is performed on a GUI button 251B "Download Answers", a file describing the input results of answers displayed for confirmation in the answer confirmation window 250, for example a JSON file, is output to the client terminal 30.

Returning to the explanation of FIG. 1, the identification unit 15B is a processing unit that identifies a flow template to be formulated and target elements to be customized on the flow template using information related to a policy accepted by the reception unit 15A. For example, the identification unit 15B receives information related to a policy and identifies a policy model composed of a plurality of components that define content related to the policy in a hierarchical structure using the received information related to the policy. More specifically, the identification unit 15B, for example, uses the received information about the policy to identify a policy model including a diagram composed of a plurality of components that define content related to the policy in a hierarchical structure.

As one aspect, the identification unit 15B identifies a flow template corresponding to the policy type for which designation was accepted by the reception unit 15A from among the flow collection data 13B. As just one example, the identification unit 15B acquires the policy type for which designation was accepted via the question window 200 illustrated in FIG. 7. For example, take the case where the item button 202B for the policy type "Regional Medical Reorganization" is selected in the question window 200 illustrated in FIG. 7. In this case, the identification unit 15B identifies the flow template f1 (see FIG. 5) in which the basic form of the policy flow of the policy type "medical function reorganization" is typified from among the flow templates included in the flow collection data 13B.

As another aspect, the identification unit 15B identifies, for each answer to a question presented by the reception unit 15A, target elements to be customized according to the answer among the specified flow templates, for example components such as services, conditional branches, etc. or parameters possessed by components. When identifying target elements in this way, related elements in which association of question templates corresponding to the answer is described among the association information 13C stored in the storage unit 13 are referenced.

The change unit 15C is a processing unit that changes target elements identified by the identification unit 15B according to a modification pattern corresponding to the answer for each answer to a question presented by the reception unit 15A. For example, the change unit 15C generates a policy model in which one or more components corresponding to information related to a policy are changed among a plurality of components possessed by the identified policy model. More specifically, for example, the change unit 15C identifies one or more components corresponding to information related to a policy among a plurality of components possessed by the identified policy model. Then, the change unit 15C generates a policy model including a diagram in which the identified one or more components are changed based on the received information about the policy.

Note that the change unit 15C may generate a policy model in which one or more components are changed using answers to questions displayed on a chat screen. For example, the reception unit 15A receives a chat start instruction from the terminal 30 and displays a question inquiring about content that customizes the identified policy model on a chat screen where the chat start instruction was accepted from the terminal 30. Then, the reception unit 15A receives answers to the questions displayed on the chat screen from the terminal 30. At this time, based on the answers to the questions, one or more components corresponding to information related to the policy among the plurality of components included in the identified policy model are changed by the change unit 15C. Then, the change unit 15C generates a policy model including a diagram in which the one or more components are changed.

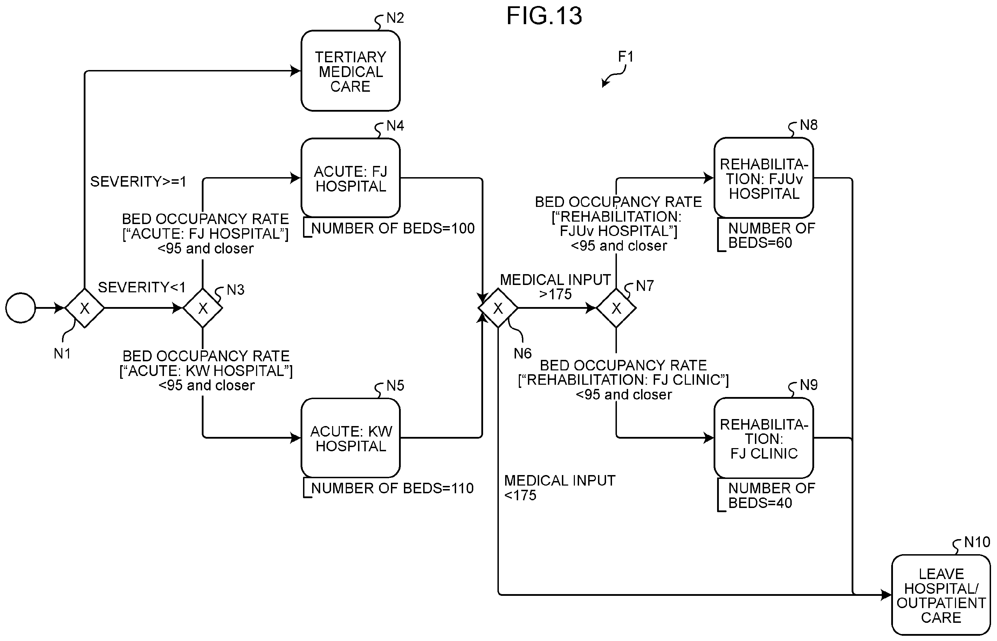

FIG. 13 is a diagram illustrating an example of a policy flow generation result. FIG. 13 illustrates a policy flow F1 generated by customizing the flow template f1 according to input results of answers exemplified in the answer confirmation window 250 illustrated in FIG. 12.

For example, according to the answer confirmation window 250 illustrated in FIG. 12, as answers to the question template "How many acute hospitals are there?" illustrated in FIG. 4, answers of two acute hospitals "FJ Hospital" and "KW Hospital" are acquired. In this case, since related element #1 describing the association of the question template How many acute hospitals are there? is referenced, components n4 and n5 corresponding to the service acute hospital in flow template f1 are identified as target elements. Furthermore, since related element #1 corresponds to modification pattern P1, the number of acute hospitals arranged in parallel in the flow template f1 is customized to two.

Also, according to the answer confirmation window 250 illustrated in FIG. 12, as answers to the question template "How many rehabilitation hospitals are there?" illustrated in FIG. 4, answers for two rehabilitation hospitals, "FJUv Hospital" and "FJ Clinic", are acquired. In this case, since related element #2 in which the association of the question template "How many rehabilitation hospitals are there?" is described is referenced, components n8 and n9 corresponding to the service "rehabilitation hospital" in the flow template f1 are identified as target elements. Furthermore, since related element #2 corresponds to modification pattern P1, the number of rehabilitation hospitals arranged in parallel in the flow template f1 is customized to two.

Furthermore, according to the answer confirmation window 250 illustrated in FIG. 12, as answers to the question template "What is the hospital name?" illustrated in FIG. 4 and the question template "Which is the medical function?" illustrated in FIG. 4, hospital name "FJ Hospital" and medical function "acute", hospital name "KW Hospital" and medical function "acute", hospital name "FJUv Hospital" and medical function "rehabilitation", and hospital name "FJ Clinic" and medical function "rehabilitation" are acquired. Since related element #3 and related element #4 in which the associations of these question templates are described are referenced, components n4 and n5 corresponding to the service "acute hospital" and components n8 and n9 corresponding to the service "rehabilitation hospital" in the flow template f1 are identified as target elements. Furthermore, related element #3 and related element #4 correspond to modification pattern P1. Therefore, each of the hospital names of components n4 and n5 corresponding to the medical function "acute" is changed to "FJ Hospital" and "KW Hospital", and each of the hospital names of components n8 and n9 corresponding to the medical function "rehabilitation" is changed to "FJUv Hospital" and "FJ Clinic".

Also, according to the answer confirmation window 250 illustrated in FIG. 12, as answers to the question template "How many beds are there?" illustrated in FIG. 4, number of beds "100" of FJ Hospital, number of beds "110" of KW Hospital, number of beds "60" of FJUv Hospital, and number of beds "40" of FJ Clinic are acquired. Since related element #5 in which the association of this question template is described is referenced, the meta-information of component n4 corresponding to FJ Hospital, the meta-information of component n5 corresponding to KW Hospital, the meta-information of component n8 corresponding to FJUv Hospital, and the meta-information of component n9 corresponding to FJ Clinic in the flow template f1 are identified as target elements. Furthermore, related element #5 corresponds to modification pattern P2. Therefore, the number of beds included in the meta-information of component n4 corresponding to FJ Hospital is changed to "100". Furthermore, the number of beds included in the meta-information of component n5 corresponding to KW Hospital is changed to "110". Furthermore, the number of beds included in the meta-information of component n8 corresponding to FJUv Hospital is changed to "60". Furthermore, the number of beds included in the meta-information of component n9 corresponding to FJ Clinic is changed to "40".

Furthermore, according to the answer confirmation window 250 illustrated in FIG. 12, as an answer to the question template "Up to what bed utilization rate can hospitalization be accepted?" illustrated in FIG. 4, the bed utilization rate "95%" is acquired. Since related element #6 in which the association of this question template is described is referenced, the bed utilization rate parameter possessed by conditional branch component n3 and the bed utilization rate parameter possessed by conditional branch component n7 in the flow template f1 are identified as target elements. Furthermore, related element #6 corresponds to modification pattern P3. Therefore, the bed utilization rate parameter possessed by conditional branch component n3 is changed to "95%", and the bed utilization rate parameter possessed by conditional branch component n7 is changed to "95%".

Also, according to the answer confirmation window 250 illustrated in FIG. 12, as an answer to the question generated from the question template "What are the stakeholders and their respective roles?" illustrated in FIG. 4, "true" for the necessity of considering tertiary emergency is acquired. Since related element #0 in which the association of this question template is described is referenced, the service "tertiary emergency" component n2 and the conditional branch component n1 that allocates objects to the service "tertiary emergency" component n2 in the flow template f1 are identified as target elements. Furthermore, related element #0 corresponds to modification pattern P4. Therefore, service tertiary emergency component n2 and conditional branch component n1 remain in the flow template f1 without being deleted. Note that although an example where "true" for the necessity of considering tertiary emergency is acquired has been given here, when "false" is acquired as the necessity of considering tertiary emergency, the service "tertiary emergency" component n2 and the conditional branch component n1 identified as target elements are deleted from the flow template f1.

By executing such a series of customizations on the flow template f1, the policy flow F1 illustrated in FIG. 13 is generated.

Returning to the explanation of FIG. 1, the output unit 15D executes various output controls. As just one example, the output unit 15D can cause the client terminal 30 to display a policy flow generated by the change unit 15C customizing the flow template. Arbitrary editing can be performed on the policy flow thus displayed on the client terminal 30 through graph editing tools and the like. Note that the output destination of the policy flow is not limited to the client terminal 30. For example, the output unit 15D can also output a policy flow generated by customization of the flow template by the change unit 15C to backend functions, services, applications, etc. operating on the above data infrastructure. For example, the output unit 15D displays the diagram of the policy model generated by the change unit 15C on the chat screen where the chat start instruction was received from the terminal 30.

### <Process Flow>

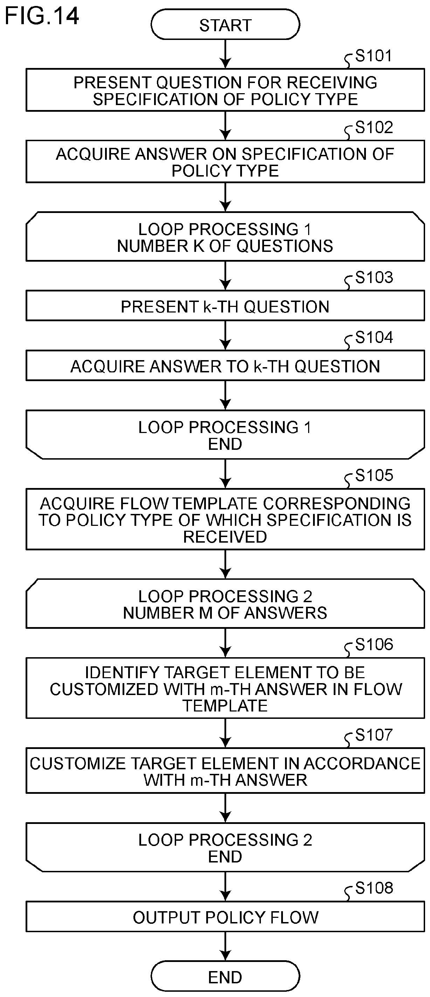

FIG. 14 is a flowchart illustrating the procedure of model output processing. This processing can be started, as just one example, when a policy flow generation request is received from the client terminal 30.

As illustrated in FIG. 14, the reception unit 15A presents to the client terminal 30 a question that accepts designation of a policy type to be formulated (step S101). Subsequently, the reception unit 15A acquires an answer for the policy type to the question presented in step S101 (step S102).

Thereafter, the reception unit 15A executes loop processing 1 by repeating steps S103 and S104 K times, corresponding to the number K of customization questions for the flow template of the policy type answered in step S102. Note that it goes without saying that steps S103 and S104 can be executed in parallel for multiple questions.

That is, the reception unit 15A presents the k-th question to the client terminal 30 based on a question template related to the policy type answered in step S102 among the question collection data 13A (step S103). Subsequently, the reception unit 15A acquires an answer to the k-th question presented in step S103 (step S104).

By repeating such loop processing 1, an answer to each question is obtained for each of the K questions.

Then, the identification unit 15B acquires the flow template corresponding to the policy type answered in step S102 from among the flow templates included in the flow collection data 13B (step S105).

Thereafter, loop processing 2 that repeats the following steps S106 and S107 for a number of times corresponding to the number K of answers obtained in loop processing 1 is executed. Note that it goes without saying that steps S106 and S107 can be executed in parallel for multiple answers.

That is, the identification unit 15B identifies a target element to be customized by the m-th answer among the flow template acquired in step S105 (step S106). Then, the change unit 15C customizes the target element identified in step S106 according to a modification pattern corresponding to the m-th answer (step S107).

By repeating such loop processing 2, customization corresponding to each answer is executed on the flow template for each of the M answers obtained. Thereby, a policy flow is generated from the flow template.

Then, the output unit 15D outputs the policy flow generated as a result of loop processing 2 to any output destination, for example, the client terminal 30 (step S108), and ends the processing.

### <One Aspect of Effects>

As described above, the server apparatus 10 according to this embodiment presents a question that customizes a type of flow in which a basic form of a policy flow is typified, and customizes a part of the type of flow based on an answer to the question. Therefore, essential components in the field are not missing, and a policy flow conforming to laws and regulations, etc. can be generated. Therefore, according to the server apparatus 10 according to this embodiment, generation of a policy model suited to the actual field where the policy is applied can be realized.

### <Second Embodiment>

Now, although embodiments related to the disclosed apparatus have been described so far, the present invention may be implemented in various different forms besides the above-described embodiments. Therefore, other embodiments included in the present invention will be described below.

### <Chat APP>

In the above First Embodiment, although an example was cited in which questions are generated based on the question collection data 13A and the questions are presented to the client terminal 30, the question collection data 13A does not necessarily need to be used for generation of questions.

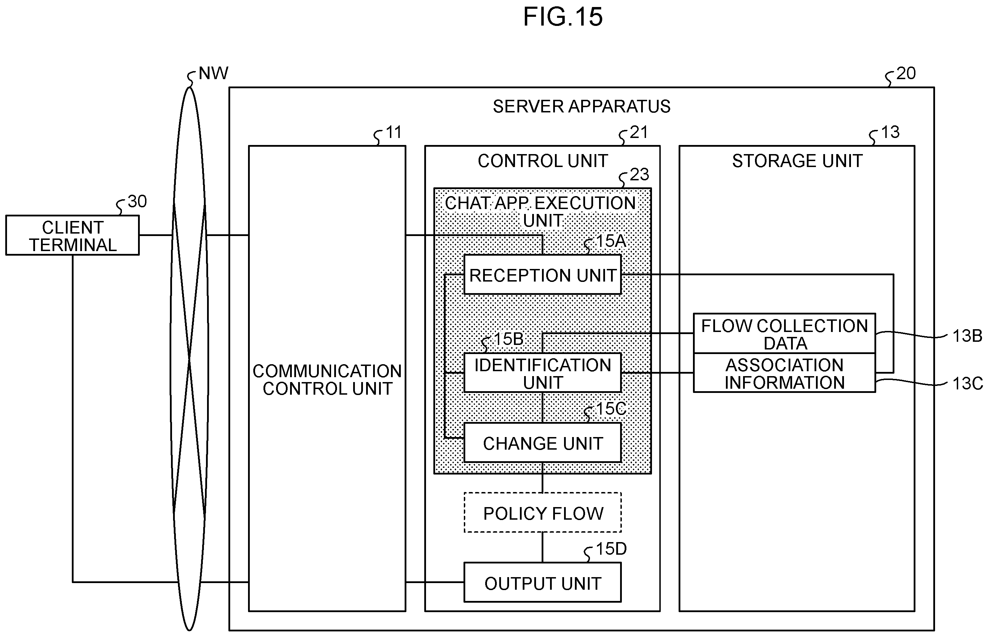

FIG. 15 is a block diagram illustrating an example of functional configuration of a server apparatus 20 according to an application example. The server apparatus 20 illustrated in FIG. 15 differs from the server apparatus 10 illustrated in FIG. 1 in that a part of the functional configuration of a control unit 21 is different, and in that the question collection data 13A does not need to be stored in the storage unit 13. Note that in FIG. 2, the same reference numerals are assigned to functional units that exhibit the same functions as those in the server apparatus 10.

As illustrated in FIG. 15, the control unit 21 has a chat APP (APPlication) execution unit 23 that exhibits the same functions as the reception unit 15A, the identification unit 15B, and the change unit 15C possessed by the control unit 15 of the server apparatus 10 illustrated in FIG. 1.

Such a chat APP execution unit 23 can be realized by executing an AI chat application program realized by a large language model such as Transformer, so-called LLM.

In this case, by providing the chat APP with a prompt for the association information 13C, the chat APP execution unit 23 can present questions equivalent to those of the reception unit 15A illustrated in FIG. 1 to the client terminal 30, even without the question collection data 13A.

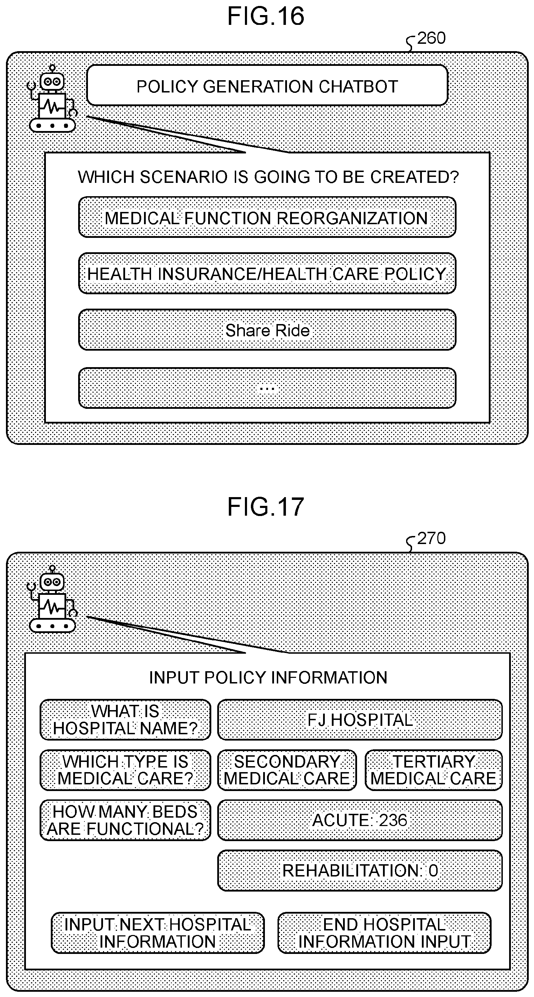

FIGS. 16 and 17 are diagrams (1) and (2) illustrating examples of a chatbot screen. FIGS. 16 and 17 illustrate chatbot screens 260 and 270 generated by the chat APP. For example, according to the chatbot screen 260 illustrated in FIG. 16, it is clear that questions equivalent to the question window 200 illustrated in FIG. 7 can be presented. Furthermore, according to the chatbot screen 270 illustrated in FIG. 17, it is clear that questions similar to the question window 210 illustrated in FIG. 8 can be presented. Note that although explanation is omitted here, chatbot screens capable of presenting equivalent questions can also be generated for the question windows 220 to 250 illustrated in FIGS. 9 to 11.

In this way, by providing a prompt for the association information 13C to the chat APP, the question collection data 13A can be made unnecessary.

### <Distribution and Integration>

Moreover, each component of each illustrated apparatus does not necessarily need to be physically configured as illustrated. That is, the specific form of distribution/integration of each apparatus is not limited to the illustrated one, and all or a part thereof can be configured by being functionally or physically distributed/integrated in arbitrary units according to various loads, usage conditions, etc. For example, the reception unit 15A, identification unit 15B, change unit 15C, or output unit 15D may be connected via a network as an external apparatus of the server apparatus 10 or server apparatus 20. Also, separate apparatuses may each have the reception unit 15A, identification unit 15B, change unit 15C, or output unit 15D, and may be network-connected and cooperate to realize the functions of the server apparatus 10 or server apparatus 20.

### <Hardware Configuration>

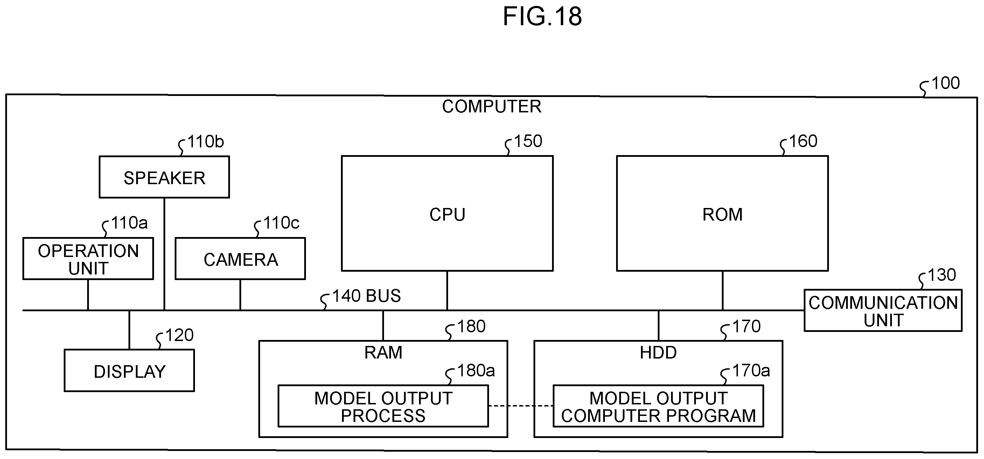

Also, the various types of processing described in the above examples can be realized by executing a prepared program on a computer such as a personal computer or workstation. Therefore, an example of a computer that executes a model output program having functions similar to Examples 1 and 2 will be described below using FIG. 18.

FIG. 18 is a diagram illustrating a hardware configuration example. As illustrated in FIG. 18, a computer 100 has an operation unit 110a, a speaker 110b, a camera 110c, a display 120, and a communication unit 130. Furthermore, this computer 100 has a CPU 150, a ROM 160, an HDD 170, and a RAM 180. These units 110 to 180 are connected via a bus 140.

The HDD 170 stores, as illustrated in FIG. 18, a model output program 170a that exhibits functions similar to the reception unit 15A, the identification unit 15B, the change unit 15C, and the output unit 15D described in First Embodiment above. This model output program 170a may be integrated or separated, similar to each component of the reception unit 15A, identification unit 15B, change unit 15C, and output unit 15D illustrated in FIG. 1. That is, not all the data illustrated in the above First Embodiment necessarily needs to be stored in the HDD 170, and it is sufficient if data used for processing is stored in the HDD 170.

Under such an environment, the CPU 150 reads out the model output program 170a from the HDD 170 and expands it to the RAM 180. As a result, the model output program 170a functions as a model output process 180a as illustrated in FIG. 18. This model output process 180a expands various data read from the HDD 170 in an area of the RAM 180 allocated to the model output process 180a, and executes various types of processing using this expanded data. For example, the processing illustrated in FIG. 14 is included as an example of processing executed by the model output process 180a. Note that in the CPU 150, not all the processing units illustrated in the above First Embodiment necessarily need to operate, and it is sufficient if the processing units corresponding to the processing to be executed are virtually realized.

Note that the above model output program 170a does not necessarily need to be stored in the HDD 170 or ROM 160 from the beginning. For example, each program is stored in a "portable physical medium" such as a flexible disk inserted into the computer 100, so-called FD, CD-ROM, DVD disk, magneto-optical disk, IC card, etc. Then, the computer 100 may acquire and execute each program from these portable physical media. Also, each program may be stored in another computer or server apparatus connected to the computer 100 via a public line, the Internet, LAN, WAN, etc., and the computer 100 may acquire and execute each program from these.

### <Third Embodiment>

Now, although in the above First Embodiment and the above Second Embodiment, an example was cited in which a basic form of a policy model collected in the flow collection data 13B is used as a template, the policy model included in the flow collection data 13B can also be used as a reference.

For example, conventional techniques for generating policy flows include prompt engineering, pre-definition of BPM-specific description rules, and PromptFlow; however, the accuracy of policy flows generated by these techniques depends entirely on user input.

According to these conventional techniques, due in part to lack of skills of users such as policy planners, only incomplete explanations can be input into prompts or definitions of BPMN, etc., and elements necessary for execution in the field may be missing from policy flows.

As one aspect, since input such as prompts and BPMN definitions depends on user skills, when user skills are insufficient, there is an aspect that explanations tend to be insufficient in user input. As a further aspect, since know-how based on personal experience and intuition is difficult to verbalize, there is also an aspect that tacit knowledge is missing from user input.

### <One Aspect of Problem-Solving Approach>

From such aspects, the model output function according to this embodiment modifies part of the user-input policy flow based on answers to questions about differences between the user-input policy flow and a reference policy flow.

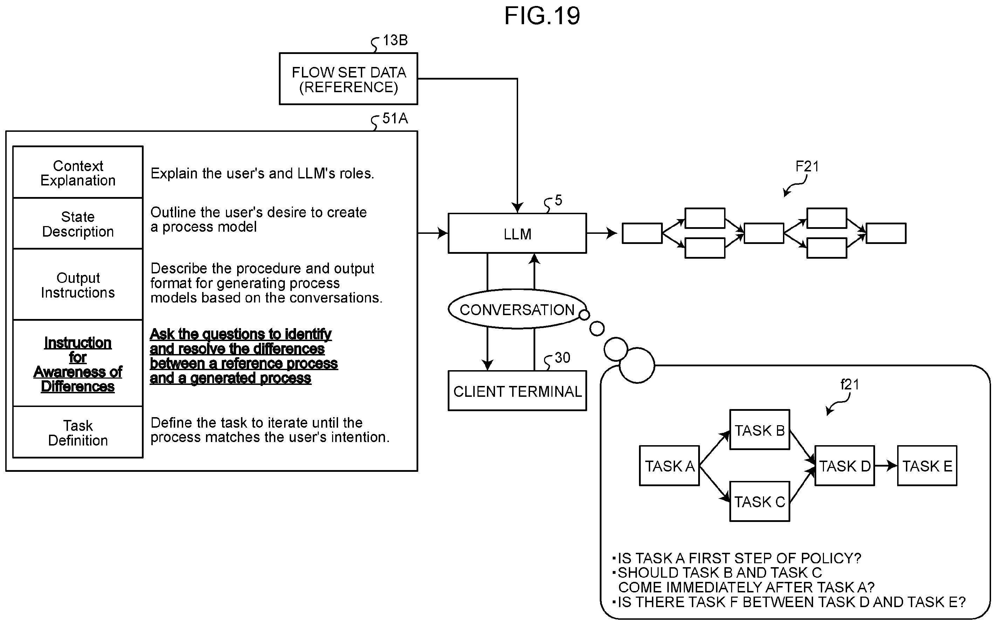

FIG. 19 is a schematic diagram illustrating an operation example of the model output function according to Example 3. As illustrated in FIG. 19, the model output function according to this embodiment can be realized by LLM 5 as an example. This LLM 5 is given, as behavioral principle data 51A, a prompt including an instruction element for generating questions based on differences from reference policy flows included in the flow collection data 13B.

For example, LLM 5 is given "Instruction for Awareness of Differences" as one prompt element, as illustrated in bold and underline in FIG. 19. This enables LLM 5 to have the following conversation with the client terminal 30.

For example, when policy flow f21 is given as user input, questions such as "Is task A the first step of the policy?", "Should task B and task C come immediately after task A?", and furthermore, "Does task F come between task D and task E?" can be presented to the client terminal 30. Note that the tasks illustrated in FIG. 19 can correspond to the "service components" or "nodes" described in the above First Embodiment.

By generating questions based on differences from reference policy flows in this way, it becomes possible to concentrate questions presented to the user on a portion of the policy.

As one aspect, the user can be guided to a state called "Selective Attention", which refers to a tendency to focus on information aligned with objectives and ignore other unrelated information even in situations of information overload. Furthermore, by repeating such questions, it is also possible to have the policy planner describe the policy while promoting their understanding.

As another aspect, compared to questions about the entire policy flow (as in Examples 1 and 2 above), it becomes possible to reduce the number of questions generated by LLM 5.

Based on the answers to these questions, LLM 5 changes part of the user-input policy flow f21. That is, by inputting answers to questions about differences from the reference, LLM 5 can generate a modified policy flow F21 in which modifications have been executed to complement elements missing from the user-input policy flow f21, for example, matters that became insufficiently explained due to the policy planner's skills or tacit knowledge.

Therefore, the model output function according to this embodiment makes it possible to generate policy flows that incorporate elements necessary for field execution.

### <Configuration of Server Apparatus 50>

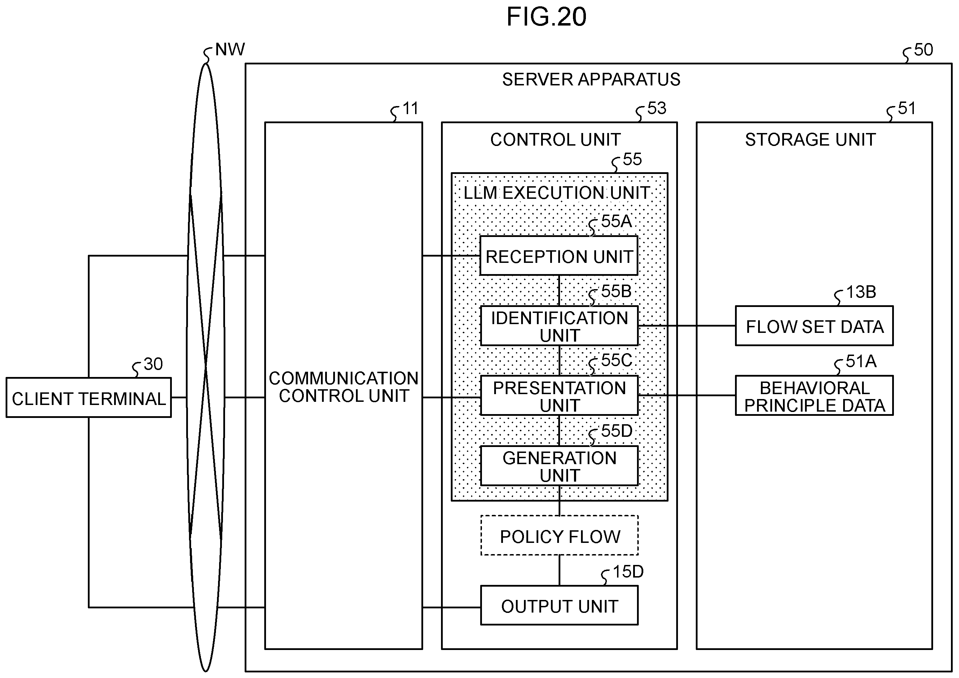

Next, the functional configuration of the server apparatus 50 that provides the model output function according to this embodiment will be described. FIG. 20 is a block diagram illustrating an example of functional configuration of the server apparatus 50 according to Example 3.

The server apparatus 50 illustrated in FIG. 20 differs from the server apparatus 10 illustrated in FIG. 1 in three respects: part of the functional configuration of a control unit 53 differs; the behavioral principle data 51A is stored in a storage unit 51; and the question collection data 13A does not need to be stored in the storage unit 51. Note that in FIG. 20, the same reference numerals are assigned to functional units that exhibit the same functions as the functional units possessed by the server apparatus 10.

As illustrated in FIG. 20, the control unit 53 has an LLM execution unit 55 that executes an LLM. This LLM execution unit 55 further has a reception unit 55A, a identification unit 55B, a presentation unit 55C, and a generation unit 55D.

The reception unit 55A, similar to the reception unit 15A illustrated in FIG. 1, receives a policy flow generation request from the client terminal 30. At this time, the reception unit 55A can also receive user input of a policy flow from the client terminal 30 together with the above policy flow generation request.

Here, the user-input policy flow may be an incomplete policy flow with errors or excesses and deficiencies, from the aspect that it is a provisional one input on the premise of being modified through the above model output function. Hereinafter, from the aspect of distinguishing the labels of both the user-input policy flow and the reference policy flow, the former may be referred to as "user flow" and the latter may be referred to as "reference flow". Furthermore, the user flow after execution of modification by the above model output function, that is, modification that changes part of the user flow, may be referred to as "modified user flow".

As just one example, the reception unit 55A can receive as a user flow a policy flow generated based on text, documents, etc. given by a user through the AI chat service mentioned in the above conventional technology. As another example, the reception unit 55A can receive as a user flow a policy flow output by the model output function described in the above First Embodiment or Second Embodiment. In addition, the reception unit 55A can also receive as a user flow a policy flow created with drawing tools that can draw flows in some format such as BPMN or mermaid by the user themselves.

The identification unit 55B is a processing unit that identifies, from among the reference flows included in the flow collection data 13B, a reference flow to compare with the user flow received by the reception unit 55A.

Here, the following policy models may be identified as examples of reference flows to be compared with the user flow. As just one example, the reference flow may be a policy model that includes all services and conditional branches of policy flows shared on the above data infrastructure platform. As another example, the reference flow may be a policy model that serves as a standard in specific domains, such as medical care, supply chain, road pricing, etc. As a further example, the reference flow may be a policy model from which common parts of multiple policy flows have been extracted. When extracting such common parts, they can be merged by applying arbitrary statistical processing to numerical values, parameters, etc. included in the common parts. As another example, the reference flow may be a minimum configuration policy model. As a further example, the reference flow may be a policy model selected via the client terminal 30 from among policy models published via the above data infrastructure platform. In addition, the reference flow may be a policy model selected based on similarity to statistical information specified via the client terminal 30, such as demographic trends, from among policy models published via the above data infrastructure platform.

The presentation unit 55C is a processing unit that presents to the client terminal 30 questions generated based on differences between the user flow received by the reception unit 55A and the reference flow identified by the identification unit 55B.

As just one example, generation of the above questions can be realized by including, as an element of the prompt in the behavioral principle data 51A, instructions to generate questions that fill the differences between the reference flow and user flow, or questions that bring the user flow closer to the reference flow.

Under such behavioral principle data 51A, the presentation unit 55C generates text based on differences between the user flow and the reference flow, presenting questions to the user in natural language using words, phrases, or sentences.

Then, the presentation unit 55C presents text corresponding to the questions to the client terminal 30. At this time, when multiple questions are generated by LLM5, the presentation unit 55C can present to the client terminal 30 a specific number (for example, any integer of 1 or more) of questions, and then sequentially present the next question each time an answer to the presented questions is obtained. In addition, the presentation unit 55C may present all questions to the client terminal 30 at once.

The generation unit 55D is a processing unit that, based on answers to questions presented by the presentation unit 55C, generates the above modified user flow by executing modifications to change one or more components among the plurality of components of the user flow received by the reception unit 55A.

Here, the above modified user flow may be graph data described in any format corresponding to BPMN, mermaid, etc. The modified user flow thus generated can be displayed on the client terminal 30 through control by the output unit 15D. In addition, from the aspect of applying the above model output function multiple times, the above modified user flow can also repeat the processing of accepting it as a new user flow for a preset number of times.

### <Process Flow>

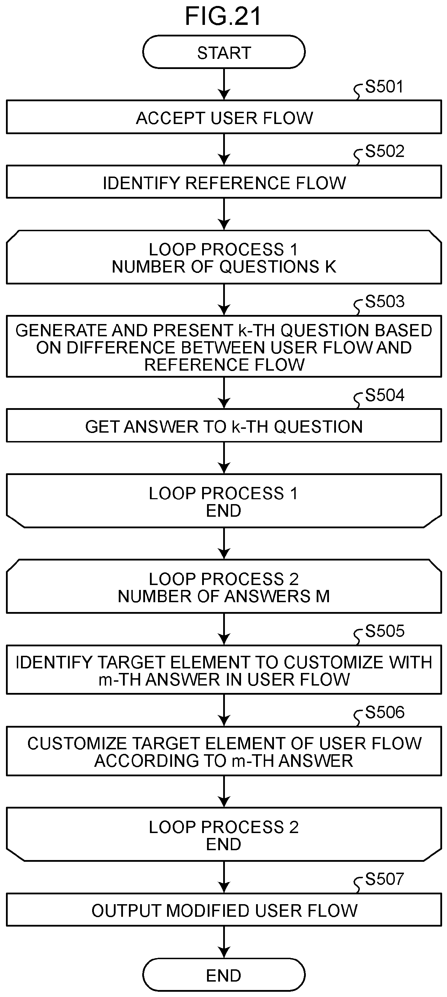

FIG. 21 is a flowchart illustrating the procedure of model output processing according to Example 3. This processing can be started, as just one example, when a policy flow generation request is received from the client terminal 30.

As illustrated in FIG. 21, the reception unit 55A receives a user flow together with the above policy flow generation request (step S501). Subsequently, the identification unit 55B identifies a reference flow to be compared with the user flow received in step S501 from among the reference flows included in the flow collection data 13B (step S502).

Thereafter, the presentation unit 55C executes loop processing 1 that repeats the following steps S503 and S504 for a number of times corresponding to the number K of questions generated based on differences between the user flow received in step S501 and the reference flow identified in step S502. Note that it goes without saying that steps S503 and S504 can be executed in parallel for multiple questions.

That is, the presentation unit 55C generates the k-th question based on differences between the user flow received in step S501 and the reference flow identified in step S502 and presents it to the client terminal 30 (step S503). Thereafter, the presentation unit 55C obtains an answer to the k-th question presented in step S503 (step S504).

By repeating such loop processing 1, an answer to each question is obtained for each of the K questions.

Then, the generation unit 55D executes loop processing 2 by repeating steps S505 and S506 for each of the M answers obtained in step S504. Note that it goes without saying that steps S505 and S506 can be executed in parallel for multiple answers.

That is, the generation unit 55D identifies target element(s) to be customized according to the m-th answer among the user flow received in step S501 (step S505). Then, the generation unit 55D customizes the target elements identified in step S505 according to the m-th answer (step S506).

By repeating such loop processing 2, customization corresponding to each answer is executed on the user flow for each of the M answers. By modifying the user flow in this way, a modified user flow is generated.

Then, the output unit 15D outputs the modified user flow generated as a result of loop processing 2 to any output destination, for example, the client terminal 30 (step S507), and ends the processing.

### <One Aspect of Effects>

As described above, the server apparatus 50 according to this embodiment modifies a part of a user-input policy flow based on answers to questions regarding differences between the user-input policy flow and a reference policy flow. Thereby, a modified policy flow in which modifications have been executed to complement elements missing from the user flow, for example, matters that became insufficiently explained due to the skills of the policy planner or tacit knowledge, can be generated. Therefore, according to the server apparatus 50 according to this embodiment, generation of a policy flow incorporating elements necessary for execution in the field can be realized.

### <Exercise of Creative Ability>

The matters described in the above Example 3, such as specific examples of large language models and description formats of graph data, are merely examples and can be changed. Also, the order of processing in the flowchart described in the above Example 3 can be changed within a range that does not cause contradictions.

### <System>

Information including processing procedures, control procedures, specific names, various data, and parameters illustrated in the above document and drawings can be arbitrarily changed except where specifically noted. For example, any one or more functional units among the LLM execution unit 55 and output unit 15D possessed by the server apparatus 50 may be configured as separate apparatuses.

For example, although an example was cited in which the reception unit 55A, the identification unit 55B, the presentation unit 55C, and the generation unit 55D are realized by executing an LLM, some of these processing units may be realized by executing software.

Also, each component of each illustrated apparatus is functionally conceptual and does not necessarily need to be physically configured as illustrated. That is, the specific form of distribution or integration of each apparatus is not limited to that illustrated. In other words, all or a part thereof can be configured by being functionally or physically distributed and integrated in arbitrary units according to various loads, usage conditions, etc. Note that each configuration may be a physical configuration.

Furthermore, each processing function performed in each apparatus may be realized in whole or in any part by a CPU (Central Processing Unit) and a program analyzed and executed by the CPU, or may be realized as hardware by wired logic.

### <Hardware>

The server apparatus 50 according to the above Example 3 can function as an information processing apparatus that executes a model output method by executing a model output program corresponding to the above model output function under the same hardware configuration as the above First Embodiment and the above Second Embodiment.

### Reference Signs List

- 10: SERVER APPARATUS
- 11: COMMUNICATION CONTROL UNIT
- 13: STORAGE UNIT
- 13A: QUESTION COLLECTION DATA
- 13B: FLOW COLLECTION DATA
- 13C: ASSOCIATION INFORMATION
- 15: CONTROL UNIT
- 15A: RECEPTION UNIT
- 15B: IDENTIFICATION UNIT
- 15C: CHANGE UNIT
- 15D: OUTPUT UNIT
- 30: CLIENT TERMINAL

## Claims

1. An output program that causes a computer to execute processing of:
receiving information related to a policy;
identifying a policy model composed of a plurality of components that prescribes content related to the policy in a hierarchical structure using the received information related to the policy;
generating a policy model in which one or more components corresponding to the information related to the policy are changed among a plurality of components possessed by the identified policy model; and
outputting the generated policy model.

2. The output program according to claim 1, wherein:
the processing of identifying the policy model identifies a policy model including a diagram composed of a plurality of components that prescribes content related to the policy in a hierarchical structure;
the processing of generating identifies one or more components corresponding to the information related to the policy among a plurality of components possessed by the identified policy model, and generates a policy model including the diagram in which the identified one or more components are changed based on the received information related to the policy; and
the processing of outputting displays the diagram of the generated policy model on a screen.

3. The output program according to claim 1, wherein:
the identified policy model is a policy model including a diagram composed of the plurality of components;
the processing of receiving includes processing of receiving a chat start instruction from a terminal, displaying on a chat screen where the chat start instruction was received from the terminal a question inquiring about content that customizes the identified policy model, and receiving from the terminal an answer to the displayed question;
the processing of generating includes processing of generating a policy model including the diagram in which the one or more components are changed based on the answer to the question; and
the processing of outputting includes processing of displaying the diagram of the generated policy model on the chat screen where the chat start instruction was received from the terminal.

4. The output program according to claim 1, wherein:
the processing of receiving includes processing of presenting questions based on question templates arranged in advance and obtaining answers to the questions;
the processing of identifying the policy model includes processing of identifying a flow template corresponding to the obtained answers among flow templates prepared in advance;
the processing of generating includes processing of identifying one or more components corresponding to the obtained answers among a plurality of components possessed by the identified flow template based on the identified flow template and association information that associates the question templates and the flow template, and generating a policy flow graph in which the one or more components among the identified flow templates are changed based on the obtained answers; and
the processing of outputting includes processing of displaying the flow graph of the generated policy flow.

5. The output program according to claim 4, further causing the computer to execute processing of accepting changes to the displayed flow graph of the policy flow.

6. The output program according to claim 4, wherein the processing of generating includes processing of changing service components arranged in parallel on the flow template or parameters possessed by the components.

7. The output program according to claim 4, wherein the processing of generating includes processing of changing meta-information of service components included in the flow template.

8. The output program according to claim 4, wherein the processing of generating includes processing of changing parameters of conditional branch components included in the flow template.

9. The output program according to claim 4, wherein the processing of generating includes processing of deleting conditional branch components included in the flow template, or conditional branch components included in the flow template and service components connected to the components.

10. The output program according to claim 1, wherein:
the processing of receiving includes processing of receiving user input of a first policy model composed of a plurality of components that prescribes content related to the policy in a hierarchical structure;
the processing of identifying includes processing of identifying a second policy model as a reference for the first policy model; and
the processing of generating includes processing of generating a third policy model by executing modifications that change one or more components among a plurality of components possessed by the first policy model based on answers to questions regarding differences between the first policy model and the second policy model.

11. The output program according to claim 10, further causing the computer to execute processing of presenting the question based on differences between the first policy model and the second policy model.

12. The output program according to claim 11, wherein the processing of presenting is realized by inputting to a large language model a prompt including elements of instructions for generating the questions based on the differences.

13. The output program according to claim 12, wherein the prompt includes instructions for generating questions that fill the differences.

14. An output method in which a computer executes processing of:
receiving information related to a policy;
identifying a policy model composed of a plurality of components that prescribes content related to the policy in a hierarchical structure using the received information related to the policy;
generating a policy model in which one or more components corresponding to the information related to the policy are changed among a plurality of components possessed by the identified policy model; and
outputting the generated policy model.

15. An information processing apparatus comprising a control unit that executes processing of:
receiving information related to a policy;
identifying a policy model composed of a plurality of components that prescribes content related to the policy in a hierarchical structure using the received information related to the policy;
generating a policy model in which one or more components corresponding to the information related to the policy are changed among a plurality of components possessed by the identified policy model; and
outputting the generated policy model.
